# EUROPEAN PATENT APPLICATION

(11) **EP 4 393 420 A1**
(43) Date of publication of application: **03.07.2024**
(21) Application number: 23200059.6
(22) Date of filing: 27.09.2023
(51) Int. Cl.: A61B 17/34, A61B 17/3209, A61B 90/00

(54) **IMPLANT DEVICE, IMPLANT ASSEMBLY, AND HOLDER**

(30) Priority: 27.09.2022 JP 2022153364
(71) Applicant: Nihon Kohden Corporation, Shinjuku-ku Tokyo (JP); University Public Corporation Osaka, Osaka-shi, Osaka 545-0051 (JP)
(72) Inventor: Makino, Hodaka, Tokorozawa-shi, Saitama (JP); Shioyama, Takahiro, Tokorozawa-shi, Saitama (JP); Nakayama, Keiko, Tokorozawa-shi, Saitama (JP); Sasaki, Tatsuya, Tokorozawa-shi, Saitama (JP); Ogawa, Tetsuya, Tokorozawa-shi, Saitama (JP); Shinozaki, Yukino, Tokorozawa-shi, Saitama (JP); Suzuki, Akane, Tokorozawa-shi, Saitama (JP); Akiyoshi, Hideo, Sakai-shi, Osaka (JP)
(74) Representative: Grünecker Patent- und Rechtsanwälte PartG mbB

(57) **Abstract**

An implant device configured to implant an implantable member into a living body includes a receiving portion having an opening surface having an opening capable of receiving the implantable member, and a support surface configured to face the opening surface in a first direction and to support the implantable member, and a discharge portion having a discharge port configured to discharge the implantable member, and a communication passage configured to communicate the discharge port and the opening and extending in a second direction intersecting with the first direction.

## Description

### TECHNICAL FIELD

The present invention relates to an implant device, an implant assembly, and a holder.

### BACKGROUND ART

A method for implanting an implantable member such as a porous body into a living body is known (for example, Patent Literature 1). Patent Literature 1 discloses a technology of implanting a porous body into a living body in order to capture circulating tumor cells (CTC).

### CITATION LIST

### PATENT LITERATURE

Patent Literature 1: US2019/0008971

### SUMMARY OF INVENTION

It is preferable that such an implantable member can be more easily implanted into a living body.

The present invention has been made in view of the above situations, and an object of the present invention is to provide an implant device, an implant assembly, and a holder capable of more conveniently implanting an implantable member into a living body.

According to an aspect of the present invention, there is provided an implant device configured to implant an implantable member into a living body. The implant device includes a receiving portion having an opening surface having an opening capable of receiving the implantable member, and a support surface configured to face the opening surface in a first direction and to support the implantable member and a discharge portion having a discharge port configured to discharge the implantable member, and a communication passage configured to communicate the discharge port and the opening and extending in a second direction intersecting with the first direction.

According to another aspect of the present invention, there is provided an implant assembly. The implant assembly includes the above implant device and a skin incision instrument having a sharp tip and scales provided at predetermined intervals from the tip.

According to another aspect of the present invention, there is provided a holder that is attachable to an implant device configured to implant an implantable member into a living body. The holder includes an accommodating space configured to accommodate the implantable member, a holding portion configured to hold the implantable member in the accommodating space, and a take-out port through which the implantable member is taken out from the accommodation space.

In the implant device and the implant assembly according to the present invention, the implantable member received from the opening passes through the communication passage and is discharged from the discharge port. In the implant device, for example, when the discharge portion is inserted subcutaneously, and a user supplies the implantable member to the receiving portion at the outside of the skin, the implantable member is discharged to a desired position under the skin. This facilitates an operation, as compared with a case where the user implants the implantable member subcutaneously using tweezers or the like. Therefore, it becomes possible to implant the implantable member more conveniently into a living body. The holder according to the present invention is mounted and used on such an implant device.

### BRIEF DESCRIPTION OF DRAWINGS

FIG. 1 is a perspective view showing a configuration of an implant assembly according to an embodiment.
FIG. 2 is a perspective view showing another state of an implant device shown in FIG. 1.
FIG. 3 is a perspective diagram showing a configuration of a body part shown in FIG 1 and the like.
FIG. 4 is a plan view showing a configuration of a side surface side of the body part shown in FIG. 3.
FIG. 5 is a plan view showing a configuration of an opening surface side of the body part shown in FIG. 3.
FIG. 6 is a view showing a cross-sectional configuration taken along a VI-VI line shown in FIG. 3.
FIG. 7 is a perspective view showing a configuration of a holder shown in FIG 1 and the like.
FIG. 8 is a perspective view showing a configuration of a back surface side of a rectangular member shown in FIG. 7.
FIG. 9 is a perspective view showing a configuration of a rod part shown in FIG 1.
FIG. 10 is a plan view showing a configuration of a side of the rod part shown in FIG. 9.
FIG. 11 is a perspective view showing a configuration of a skin incision instrument shown in FIG. 1.
FIG. 12 is a plan view showing a configuration of a side surface side of the skin incision instrument shown in FIG. 11.

### DESCRIPTION OF EMBODIMENTS

Hereinafter, an embodiment concerning one aspect of the present invention will be described. The present invention is not limited only to the following embodiment.

### <Embodiment>

### [Configuration of Implant Assembly]

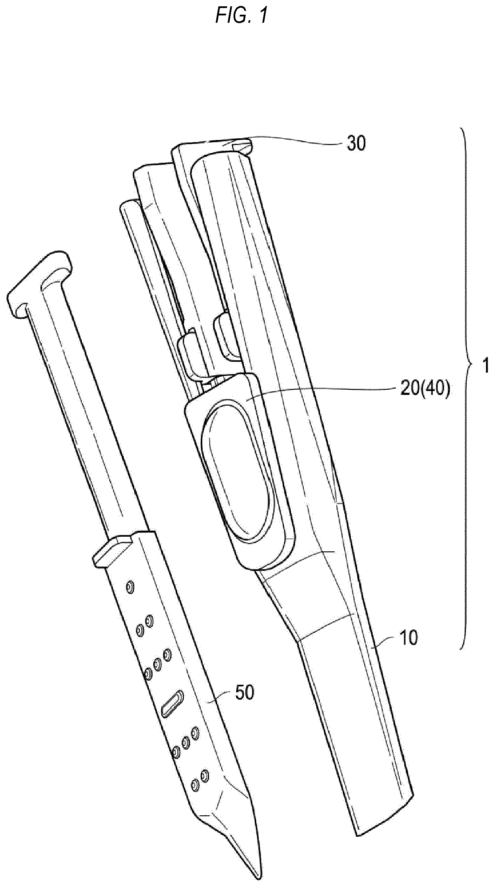

FIG. 1 shows an example of a configuration of an implant assembly according to an embodiment of the present invention. The implant assembly includes, for example, an implant device 1 and a skin incision instrument 50. The implant device 1 is partially inserted under the skin of a living body, and discharges an implantable member (an implantable member 40 described below) into the living body. The implant device 1 includes, for example, a body part 10, a holder 20, and a rod part 30, and the implantable member 40 is held in the holder 20.

The skin incision instrument 50 is used, for example, before inserting the implant device 1 subcutaneously. Auser such as a doctor or a veterinarian forms a pocket for implanting the implantable member 40 under the skin of a living body using, for example, the skin incision instrument 50.

The implant assembly may be used for implanting the implantable member 40 in humans, or may be used for implanting the implantable member in animals such as dogs, cats, rabbits, mice, and pigs.

The implantable member 40 is, for example, a porous body having a cancer cell capturing function. Cancer cell capturing means that a cancer cell adheres to a side surface of the porous body or inside a pore thereof. A porous body means a solid material or a structure including a plurality of pores. The porous body includes, for example, an inorganic material such as ceramic. The implantable member 40 has communication pores on a surface of the porous body for cancer cell capturing, for example. The communication pores are pores where adjacent pores are connected to each other. Therefore, when cancer cells enter the pores of the porous body, more cancer cells can adhere thereto. That is, more cancer cells can be captured. It is preferable that the implantable member 40 has, for example, fine irregularities on its surface, and thus a surface area of the implantable member 40 is made large. The implantable member 40 is, for example, a fragile member, and is likely to be damaged by impact such as vibration.

A kind of cancer captured by the implantable member 40 is not particularly limited. Examples thereof may include cancers of the cranial nervous system (e.g., brain tumor, schwannoma, meningioma, hypophyseal adenoma, spinal cord tumor); cancers of the head and neck (oral cancer, pharyngeal cancer, larynx cancer, thyroid cancer, parotid gland cancer); cancer of the digestive system (e.g. esophageal cancer, stomach cancer, liver cancer, gallbladder cancer, biliary tract cancer, spleen cancer, large intestine cancer, small intestine cancer, duodenal cancer, colon cancer, colon adenocarcinoma, rectal cancer, pancreatic cancer, hepatic cancer); cancers of the musculoskeletal system (e.g., sarcoma, osteosarcoma, myeloma); cancers of the urinary system (e.g., bladder cancer, kidney cancer); cancers of the reproductive system (e.g., breast cancer, uterine cancer, ovarian cancer, testicular cancer, prostate cancer); cancers of the respiratory system (e.g., lung cancer); cancers of the hematopoietic system (e.g., leukemia such as acute or chronic myeloid leukemia, acute promyelocytic leukemia, and acute or chronic lymphocytic leukemia, malignant lymphoma (lymphosarcoma), angiosarcoma, multiple myeloma, myelodysplastic syndrome, primary myelofibrosis, hemangiopericytoma); thyroid cancer, parathyroid cancer, malignant melanoma (melanoma), mast cell tumor, cutaneous histiocytoma, lipoma, follicular tumor, cutaneous papilloma, sebaceous adenoma, basal cell carcinoma, and the like.

The cancer cells captured by the implantable member 40 are, for example, metastatic cancer cells. The metastatic cancer cells are not particularly limited, and are, for example, metastatic cancer cells originating from the above cancers, preferably metastatic cancer cells originating from breast cancer, pancreatic cancer, lung cancer, hepatic cancer, and brain tumor.

A shape and a size of the implantable member 40 are not particularly limited, and can be appropriately selected according to the usage, the purpose of use, the site of use, and the like. The implantable member 40 has, for example, an elliptical plane shape. The implantable member 40 may have a circular plane shape, a spherical shape, or a hemispherical shape.

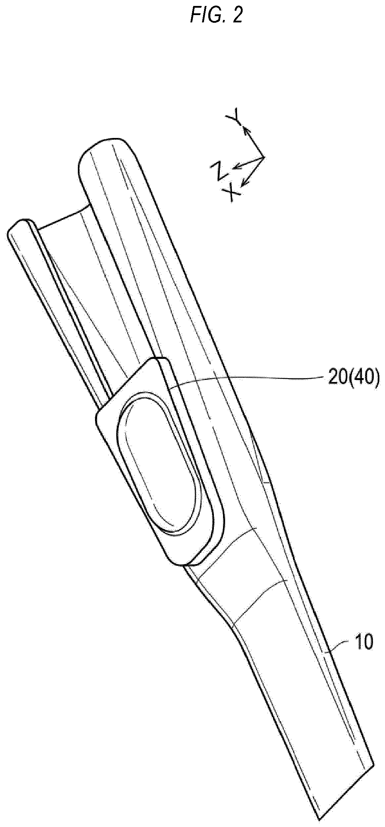

FIG. 2 shows a state in which the rod part (rod part 30) is removed from the implant device 1 shown in FIG. 1. In this way, the implant device 1 may not be provided with the rod part.

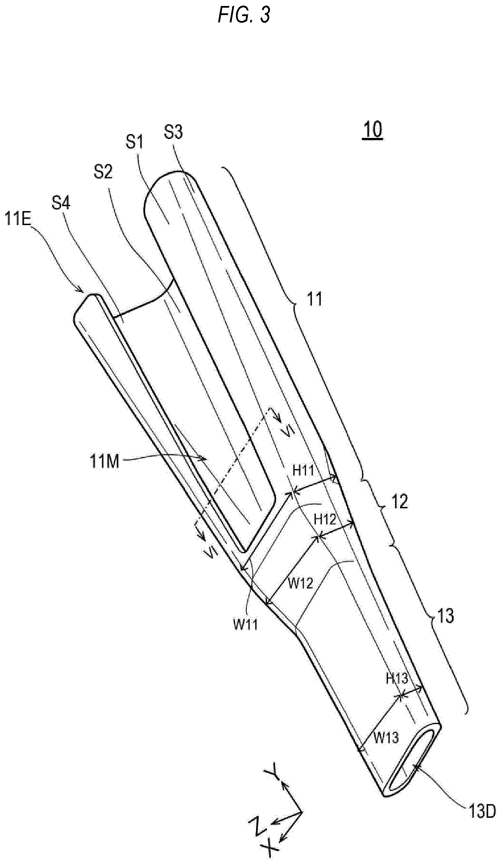

FIG. 3 is a perspective view showing a configuration of the body part 10 of the implant device 1. The body part 10 has a nozzle shape surrounded by an opening surface S1, a support surface S2, and a pair of side surfaces S3 and S4, and extends in a predetermined direction. The body part 10 has a receiving portion 11, a connecting portion 12, and a discharge portion 13 in this order along the extension direction. An open end 11E is provided at an end portion on the receiving portion 11 side, and a discharge port 13D is provided at an end portion on the discharge portion 13 side. In the following description, the extension direction of the body part 10 may be referred to as a Y direction (second direction), a width direction of the body part 10 may be referred to as an X direction (third direction), and a height direction of the body part 10 may be referred to as a Z direction (first direction). The body part 10 is made of, for example, a biocompatible resin material or the like.

The opening surface S1 is a surface substantially parallel to the XY plane. The opening surface S1 may be a flat surface or a curved surface. The opening surface S1 is provided with an opening 11M that can receive the implantable member 40. The holder 20 is attached to the opening 11M.

The support surface S2 faces the opening surface S1 in the Z direction, and supports the implantable member 40 taken out from the holder 20. The support surface S2 is preferably a smooth surface. Thereby, the implantable member 40 can easily move on the support surface S2.

The pair of side surfaces S3 and S4 connect the opening surface S1 and the support surface S2. The side surface S3 and the side surface S4 are provided facing each other in the X direction. The opening surface S1, the support surface S2, and the side surface S3, S4 are provided over the receiving portion 11, the connecting portion 12, and the discharge portion 13.

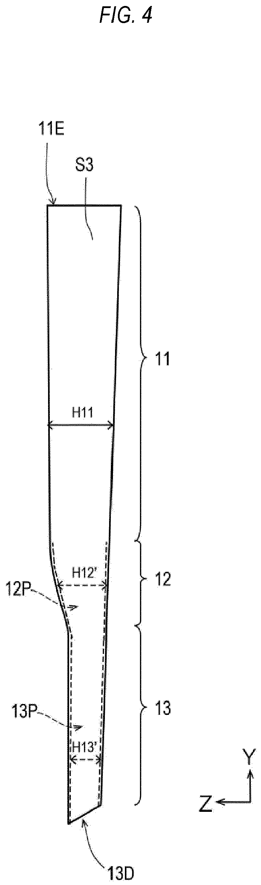

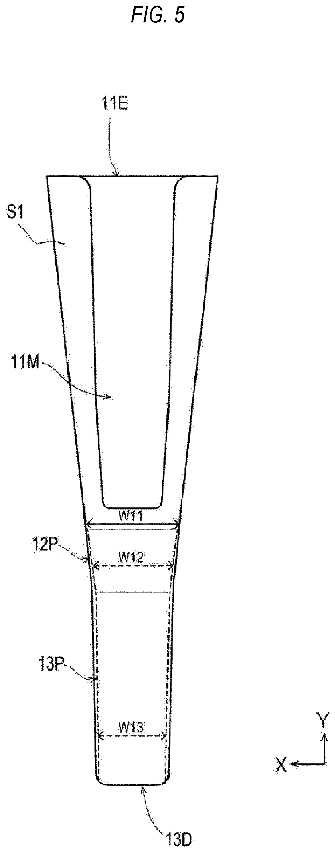

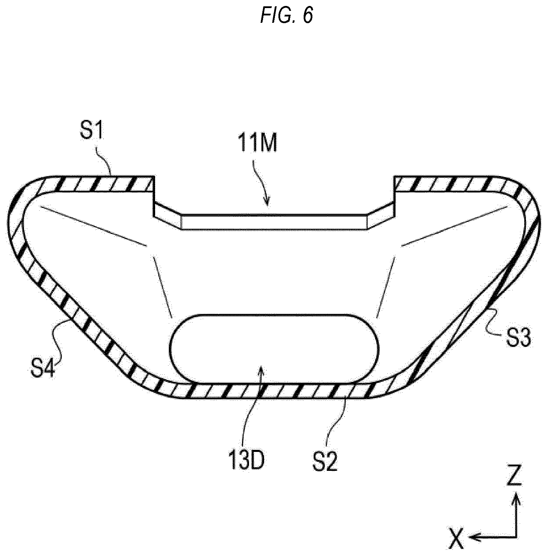

FIG. 4 shows a plane (YZ plane) configuration on the side surface S3 side of the body part 10, FIG. 5 shows a plane (XY plane) configuration on the opening plane S1 side, and FIG. 6 shows a configuration of a cross section (XZ cross section) taken along a line VI-VI of FIG. 3. Hereinafter, each configuration of the receiving portion 11, the connecting portion 12, and the discharge portion 13 is described using FIGS. 4 to 6, together with FIG. 3.

The receiving portion 11 is a portion for receiving the implantable member 40 taken out from the holder 20. The receiving portion 11 is gripped by a user's hand, for example. The receiving portion 11 is provided with the opening 11M. The opening 11M has, for example, a rectangular plane shape (FIG. 5), and is formed from the vicinity of the connecting portion 12 to the open end 11E. That is, the opening 11M connects to the open end 11E, and the holder 20 can be attached and detached with respect to the opening 11M from the open end 11E side.

The receiving portion 11 has a width W11 in the X direction and a height H11 in the Z direction (FIGS. 3 to 5). In the receiving portion 11, the opening surface S1 has a substantially trapezoidal plane shape (FIG. 5), and the width W11 gradually decreases from the open end 11E toward the connecting portion 12. The height H11 is substantially the same from the open end 11E to the connecting portion 12, for example. The height H11 may decrease gradually or stepwise from the open end 11E toward the connecting portion 12.

The receiving portion 11 has, for example, a substantially trapezoidal cross section (XZ cross section) shape (FIG. 6), and at a predetermined position in the Y direction, a size (width W11) in the X direction of the opening surface S1 is larger than a size in the X direction of the support surface S2. This makes it possible to increase the size in the X direction of the opening 11M, and to take out the implantable member 40 smoothly.

The connecting portion 12 provided between the receiving portion 11 and the discharge portion 13 has a connecting passage 12P (FIG. 4 and FIG. 5). The connecting passage 12P is a passage for the implantable member 40 surrounded by the opening surface S1, the support surface S2, and the side surfaces S3 and S4, and connects the opening 11M and the discharge port 13D (specifically, a communication passage 13P described below).

The connecting portion 12 has a width W12 in the X direction and a height H12 in the Z direction (FIG. 3). In the connecting portion 12, the opening surface S 1 has a substantially trapezoidal plane shape (FIG. 5), and the width W12 gradually decreases from the receiving portion 11 side toward the discharge portion 13 side. The height H12 gradually decreases from the receiving portion 11 side toward the discharge portion 13 side. A width W12' in the X direction and a height H12' in the Z direction of the connecting passage 12P are substantially the same as the width W12 and the height H12, respectively, and gradually decrease from the receiving portion 11 side toward the discharge portion 13 side. Since the connecting passage 12P has such width W12' and height H12', the major axis direction of the substantially elliptical implantable member 40 is aligned in the extension direction (Y direction) of the connecting passage 12P between the receiving portion 11 and the discharge portion 13, allowing the implantable member 40 to be oriented without applying an excessive force. That is, it becomes possible to implant the implantable member 40 subcutaneously in a desired direction.

The discharge portion 13 is a portion that is inserted under the skin of a living body, and discharges the implantable member 40 to a desired location under the skin. The discharge portion 13 has a discharge port 13D and a communication passage 13P. The discharge port 13D is arranged, for example, in a pocket formed under the skin and discharges the implantable member 40. The communication passage 13P is a passage for the implantable member 40 surrounded by the opening surface S1, the support surface S2, and the side surfaces S3 and S4, and connects the connecting passage 12P and the discharge port 13D.

The discharge portion 13 has a width W13 in the X direction and a height H13 in the Z direction (FIG. 3). In the discharge portion 13, the opening surface S1 has a rectangular plane shape (FIG. 5), and the width W13 has substantially the same size from the connecting portion 12 side to the discharge port 13D, for example. The height H13 also has substantially the same size from the connecting portion 12 side to the discharge port 13D. The sizes of the width W13 and the height H13 are set smaller than the minimum width W11 and the minimum height H13 of the receiving portion 11 (specifically, the width W11 and the height H13 at an end portion on the connection portion 12 side) (W11>W13, H11>H13). A width W13' in the X direction and a height H13' in the Z direction of the communication passage 13P are substantially the same as the width W13 and the height H13, respectively, and have substantially the same sizes from the connecting portion 12 side to the discharge port 13D. The size of the width W13' is slightly larger than a size of a minor axis of the implantable member 40 having, for example, an elliptical plane shape, and is also smaller than a size of a major axis of the implantable member 40. The size of the height H13' is slightly larger than a size in the thickness direction of the implantable member 40, for example. In this way, since the communication passage 13P has an appropriate size corresponding to the shape of the implantable member 40, a pocket for implanting the implantable member 40 does not need to be larger than necessary. That is, it is possible to reduce the burden on the living body and to realize low-invasive implanting.

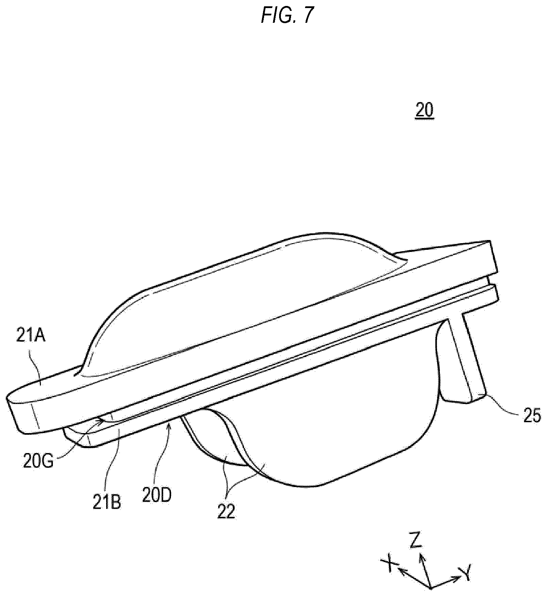

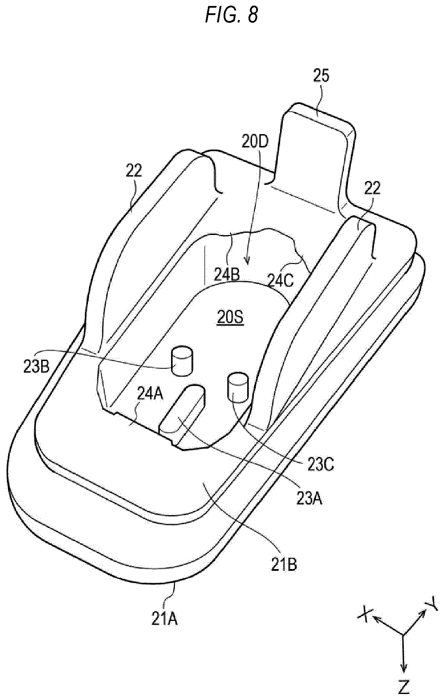

FIGS. 7 and 8 are perspective views showing a configuration of the holder 20. FIG. 8 shows a state in which the holder 20 shown in FIG. 7 is inverted in the Z direction. The holder 20 serves to protect the fragile implantable member 40 from an external impact. For example, the implantable member 40 is carried to the user in a state of being held by the holder 20. The holder 20 is preferably made of a biocompatible resin material having flexibility, such as a soft elastomer, for example.

The holder 20 attached to the opening 11M of the body part 10 has, for example, a substantially rectangular plane (XY plane) shape. The holder 20 has, for example, a rectangular member 21A forming an accommodating space 20S, a frame member 21B having a take-out port 20D, a falling-off prevention portion 22 extending from the frame member 21B, protrusions 23A, 23B and 23C (second protrusion) provided on the rectangular member 21A, protrusions 24A, 24B and 24C (first protrusion) provided on the frame member 21B, and a stopper 25 provided at an end of the holder 20 in a long side direction. Here, the protrusions 23A, 23B and 23C and the protrusions 24A, 24B and 24C correspond to one specific example of the holding portion of the present invention. The holder 20 is attached to the body part 10 such that a long side of the rectangular member 21A is along the Y axis and the stopper 25 is arranged on the open end 11E side of the body part 10.

The rectangular member 21A has a convex portion on its surface, and the accommodating space 20S is formed on a back surface side of the rectangular member 21A by the convex portion. The back surface of the rectangular member 21A is arranged facing the support surface S2 of the body part 10. The accommodating space 20S (convex portion) has, for example, a substantially elliptical plane (XY plane) shape. The implantable member 40 is accommodated in the accommodating space 20S. The convex portion on the surface of the flexible rectangular member 21A, i.e., a wall of the accommodating space 20S is configured to be deformable toward the take-out port 20D side. Thereby, when the user presses the convex portion on the surface of the rectangular member 21A in the Z direction, the accommodating space 20S and the protrusions 23A, 23B, 23C, 24A, 24B and 24C are deformed, and the implantable member 40 held in the accommodating space 20S is taken out from the take-out port 20D.

The protrusions 23A, 23B and 23C protrude from the back surface of the rectangular member 21A, more specifically, from the wall of the accommodating space 20S toward the take-out port 20D side, and are in contact with the implantable member 40 accommodated in the accommodating space 20S. For example, the protrusion 23A has an elliptical plane (XY plane) shape, and the protrusions 23B and 23C each have a circular plane shape. For example, the protrusion 23A and the protrusions 23B and 23C are arranged at different positions in the Y direction. The protrusion 23A is arranged at a position farther from the stopper 25 in the Y direction than the protrusions 23B and 23C. The protrusion 23B and the protrusion 23C are the same in position in the Y direction, for example, and are arranged side by side in the X direction. The number, shape and arrangement of the protrusions 23A, 23B and 23C are not limited to the above.

The frame member 21B is provided in a frame shape at an edge of the accommodating space 20S, and has the take-out port 20D at a position overlapping the accommodating space 20S. The implantable member 40 held in the accommodating space 20S is taken out to the receiving portion 11 of the body part 10 via the take-out port 20D. The take-out port 20D has, for example, substantially the same plane shape as the accommodating space 20S, and has, for example, a substantially elliptical plane shape.

The protrusions 24A, 24B, and 24C protrude from an edge of the take-out port 20D toward the take-out port 20D, and are in contact with the implantable member 40 accommodated in the accommodating space 20S. The protrusion 24A and the protrusions 24B and 24C are arranged facing each other in the major axis direction (Y direction) of the elliptical take-out port 20D. The protrusions 24B and 24C are arranged at positions closer to the stopper 25 in the Y direction than the protrusion 24A. The implantable member 40 is held between the protrusions 24A, 24B and 24C and the protrusions 23A, 23B and 23C. The number, shape and arrangement of the protrusions 24A, 24B and 24C are not limited to the above.

A groove 20G extending in the Y direction is provided between the rectangular member 21A and the frame member 21B. The groove 20G is capable of fitting into an edge of the opening 11M of the body part 10. When the user inserts the groove 20G into the edge of the opening 11M, the holder 20 is attached to the body part 10 (receiving portion 11), and when the user pulls out the groove 20G from the edge of the opening 11M, the holder 20 is separated from the body part 10. In this way, the holder 20 is configured to be attached and detached with respect to the body part 10. This makes it easy to attach the implantable member 40 to the holder 20.

The falling-off prevention portion 22 serves to prevent the groove 20G from falling off from the edge of the opening 11M when the implantable member 40 is taken out from the take-out port 20D. The falling-off prevention portion 22 is attached to the frame member 21B, for example. For example, a pair of falling-off prevention portions 22 are provided so as to face each other in the minor axis direction (X direction) of the elliptical take-out port 20D. The falling-off prevention portion 22 is composed of, for example, a substantially rectangular plate-like member, and a short side of the plate-like member is arranged in the Z direction, and a long side is arranged in the Y direction. That is, the falling-off prevention portion 22 has predetermined lengths in the Z direction and the Y direction, respectively. When the holder 20 is attached to the body part 10, the falling-off prevention portion 22 is arranged between the opening surface S1 and the support surface S2 of the receiving portion 11. As a result, when the user presses the convex portion on the rectangular member 21A in the Z direction, the falling-off prevention portion 22 comes into contact with the support surface S2 before the groove 20G falls off from the edge of the opening 11M, so that it becomes difficult for the groove 20G to fall off from the edge of the opening 11M.

The stopper 25 serves to prevent the implantable member 40 dropped from the take-out port 20D onto the support surface S2 of the body part 10 from moving toward a side opposite to the discharge port 13D, i.e., toward the open end 11E side. The stopper 25 is, for example, attached to an end of the frame member 21B in the Y direction, and is integrated with the holder 20. The stopper 25 is composed of, for example, a substantially rectangular plate-like member, and a short side of the plate-like member is arranged in the X direction, and a long side is arranged in the Z direction. That is, the stopper 25 has predetermined lengths in the X direction and the Z direction, respectively. The stopper 25 is provided between the opening surface S1 and the support surface S2 and is arranged at a position facing the discharge port 13D with the take-out port 20D interposed therebetween, when the holder 20 is attached to the body part 10. That is, the stopper 25 is arranged to block the open end 11E. Thereby, even when the implantable member 40 taken out from the take-out port 20D moves toward the open end 11E side, the implantable member comes into contact with the stopper 25 and is likely to return to the discharge port 13D side. The flexible stopper 25 is configured to be deformable in response to an applied force.

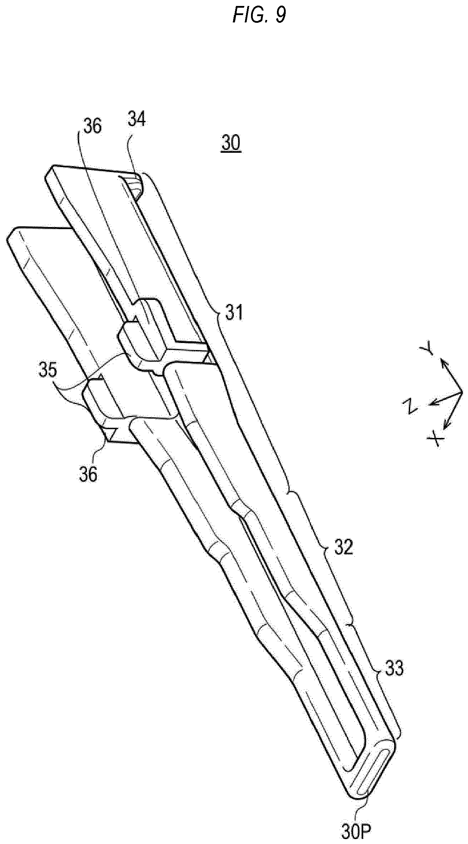

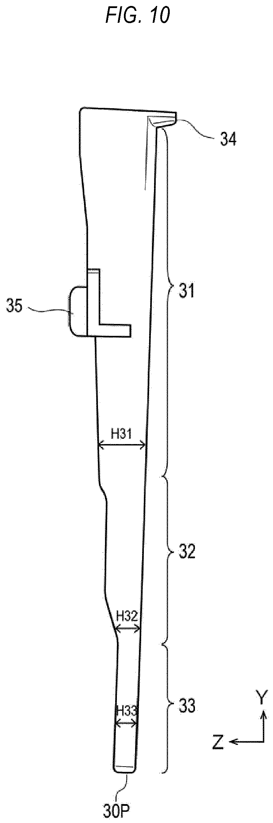

FIGS. 9 and 10 show a configuration of the rod part 30. FIG. 9 is a perspective view showing the configuration of the rod part 30, and FIG. 10 is a plan view showing the configuration of a side surface (YZ plane) of the rod part 30. The rod part 30 is inserted between the opening surface S1 and the support surface S2 of the body part 10 and serves to push the implantable member 40 toward the discharge port 13D side when the implantable member 40 does not move smoothly toward the discharge port 13D.

The rod part 30 extends in a predetermined direction and is inserted into the body part 10 so that the extension direction is along the extension direction (Y direction) of the body part 10. The rod part 30 has, for example, a base portion 31, an intermediate portion 32, and a tip portion 33 in this order along the Y direction, and an end of the tip portion 33 is provided with a tip 30P. The rod part 30 further has a regulating portion 34, a protruding portion 35, and a wide portion 36 provided on the base portion 31. The rod part 30 is made of, for example, a biocompatible resin material, a sterilizable metal material or the like.

The base portion 31, the intermediate portion 32, and the tip portion 33 are arranged in the receiving portion 11, the connecting portion 12, and the discharge portion 13, respectively, when the rod part 30 is inserted into the body part 10. The base portion 31, the intermediate portion 32, and the tip portion 33 have heights H31, H32, and H33 in the Z direction, respectively.

The height H31 of the base portion 31 is smaller than the height H11 of the receiving portion 11. It is preferable that, when the holder 20 is attached to the body part 10, the base portion 31 is in contact with the protrusions 24A, 24B and 24C of the holder 20. This makes it difficult for the holder 20 to fall off from the body part 10.

The height H32 of the intermediate portion 32 has a portion that gradually decreases from the base portion 31 side toward the tip portion 33 side, for example. When the rod part 30 is inserted into the body part 10, at a predetermined position in the Y direction, the height H32 is smaller than the height H12' of the connecting passage 12P.

The height H33 of the tip portion 33 is smaller than the height H13' of the communication passage 13P. The height H33 is smaller than the height H31 (H31 >H33). When the user inserts the rod part 30 into the body part 10 to which the holder 20 is attached, the tip 30P pushes up the stopper 25 and advances toward the discharge port 13D. The rod part 30 is configured such that at least the tip 30P is insertable into a part of the communication passage 13P via a gap between the opening surface S1 and the support surface S2 of the body part 10.

Although not shown, sizes in the X direction of the base portion 31, the intermediate portion 32 and the tip portion 33 are smaller than the widths W11, W12' and W13' of the receiving portion 11, the connecting passage 12P, and the communication passage 13P, respectively.

The regulating portion 34 is provided at an end opposite to the tip 30P in the Y direction, and protrudes from the base portion 31 in the Z direction. When the rod part 30 is inserted into the body part 10, the regulating portion 34 is caught on the support surface S2 at the open end 11E and is not inserted into the body part 10. That is, since a position of the tip 30P of the rod part 30 in the Y direction is regulated to a position at the time when the regulating portion 34 reaches the open end 11E, the tip 30P can be suppressed from protruding from the discharge port 13D.

The protruding portion 35 protrudes from the base portion 31 in the Z direction. The base portion 31 is provided with a pair of protruding portions 35 facing each other in the X direction, for example. For example, when the user shifts the rod part 30 inserted into the body part 10 in the X direction, the protruding portions 35 are in contact with the opening surface S 1, so that it is difficult for the rod part 30 to separate from the opening 11M of the body part 10.

The wide portion 36 is wide in the X direction from the base portion 31. The base portion 31 is provided with a pair of wide portions 36 facing each other in the X direction, for example. For example, when the rod part 30 inserted into the body part 10 swings in the Z direction, the wide portions 36 are in contact with the side surfaces S3 and S4, so the user can easily insert the rod part 30 along the extension direction of the body part 10.

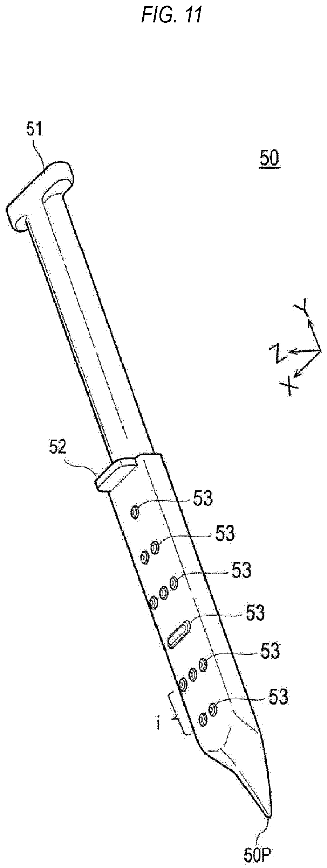

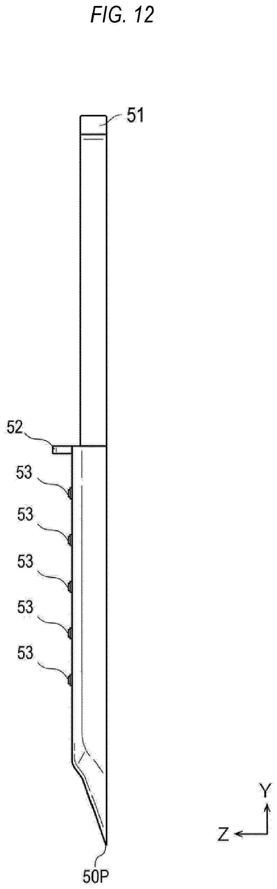

FIGS. 11 and 12 show a configuration of the skin incision instrument 50. FIG. 11 is a perspective view showing the configuration of the skin incision instrument 50, and FIG. 12 is a plan view showing the configuration of a side surface (YZ plane) of the skin incision instrument 50. The skin incision instrument 50 has, for example, a stick shape having a front surface and a back surface (XY plane) and extending in the Y direction. A tip 50P in the Y direction has a sharp blade shape. The user forms a pocket for implanting the implantable member 40 under the skin of a living body using the tip 50P. The skin incision instrument 50 has, for example, a gripping portion 51, a partition 52, and scales 53, in addition to the tip 50P.

The gripping portion 51 is provided at an end opposite to the tip 50P, for example, and has a wide portion in the X direction of the skin incision instrument 50. By providing the skin incision instrument 50 with such a gripping portion 51, the user can easily hold the skin incision instrument 50.

The partition 52 is arranged, for example, near a center of the skin incision instrument 50 in the Y direction, and is erected from the surface in the Z direction. By providing such a partition 52 on the surface of the skin incision instrument 50, the user can easily determine the front and rear surfaces of the skin incision instrument 50.

The plurality of scales 53 are provided on the front surface of the skin incision instrument 50, for example, and are arranged at predetermined intervals i between the partition 52 and the tip 50P. By providing the plurality of scales 53 on the skin incision instrument 50, the user can easily measure a depth of the pocket formed under the skin. Therefore, a pocket having a depth suitable for the implantable member 40 can be easily formed. The plurality of scales 53 may be displayed differently depending on their positions, for example. For example, the scale 53 at a position of 4 cm from the tip 50P is displayed differently from the other scales 53. This makes it easier to check the depth of the pocket.

### [Use Method of Implant Assembly]

Next, a use method of the implant assembly of the present embodiment will be described.

First, the user forms a pocket of an appropriate depth under the skin of a living body using the skin incision instrument 50. The user may form a pocket using an instrument other than the skin incision instrument 50.

Next, the user grips the receiving portion 11 of the body part 10 to which the holder 20 is attached, and inserts the discharge portion 13 into the pocket. Here, in the present embodiment, since the width W11 and height H11 of the receiving portion 11 are larger than the width W13 and height H13 of the discharge portion 13, it is possible to prevent the user from excessively inserting the body part 10 subcutaneously. Therefore, the burden on the living body can be reduced. In addition, the larger receiving portion 11 enables the user to easily grip the same ergonomically, and its handling is thus facilitated. In the holder 20 attached to the body part 10, the implantable member 40 is held in the accommodating space 20S.

The user inserts the discharge port 13D of the body part 10 to a desired position in the pocket formed under the skin, and then presses the convex portion on the surface of the rectangular member 21A of the holder 20 in the Z direction at the outside of the skin of the living body. Thereby, the accommodating space 20S is deformed, and the protrusions 23A, 23B, 23C, 24A, 24B and 24C are also deformed, so that the implantable member 40 falls onto the support surface S2 via the take-out port 20D. For example, the user can supply the implantable member 40 held by the holder 20 to the body 10 by an operation with one hand.

Next, the user tilts the body part 10 so that the receiving portion 11 is higher than the discharge portion 13. Thereby, the implantable member 40 that has fallen on the support surface S2 slidingly moves on the smooth support surface S2 with its own weight. At this time, since the width W12' and the height H12' of the connecting passage 12P gradually decrease toward the communication passage 13P, the implantable member 40 is oriented. Specifically, the major axis of the substantially elliptical implantable member 40 aligns with the extension directions of the connecting passage 12P and the communication passage 13P, and the implantable member is implanted into the subcutaneous pocket from the discharge port 13D. In the case where the implantable member 40 stays in the body part 10, the user inserts the rod part 30 into the body part 10 and moves the implantable member 40 to the discharge port 13D by the tip 30P.

### [Operational Effects of Implant Assembly]

In the implant device 1 and the implant assembly according to the present embodiment, the implantable member 40 received through the opening 11M passes through the communication passage 13P and is discharged from the discharge port 13D. As described above, in the implant device 1, for example, when the discharge portion 13 is inserted subcutaneously and the user supplies the implantable member 40 to the receiving portion 11 from the outside of the skin, the implantable member 40 is discharged to a desired position under the skin. This facilitates an operation, as compared with a case where the user implants the implantable member subcutaneously using tweezers or the like. Hereinafter, the operational effects will be described.

When the user implants an implantable member subcutaneously using tweezers or the like, appropriate implantation may be difficult. For example, when fine irregularities are provided on the surface of the implantable member 40, the subcutaneous tissue is likely to be entangled with the fine irregularities, making appropriate implantation difficult.

In contrast, in the implant device 1, the user supplies the implantable member 40 to the body part 10 from the outside of the skin, so that the implantable member 40 is implanted to a desired position from the discharge port 13D. Therefore, even when the surface of the implantable member 40 has fine irregularities, it is possible to easily implant the implantable member 40 without using tweezers or the like.

In addition, the fragile implantable member 40 may be damaged if gripped with tweezers or the like. However, by using the implant device 1, even the fragile implantable member 40 can be safely implanted while suppressing occurrence of the damage. Further, by holding the implantable member 40 in the holder 20, it is possible to implant the implantable member 40 more safely.

Additionally, in the implant device 1, the implantable member 40 can be easily implanted to a desired position by arranging the discharge port 13D at an appropriate position under the skin. Therefore, it is possible to reliably implant the implantable member 40 subcutaneously.

Further, in the case where the user implants the implantable member into the subcutaneous pocket using tweezers or the like, it is necessary to form a slightly larger pocket with a margin. However, in the implant device 1, a pocket having such a size that the discharge portion 13 can be inserted just needs to be formed, which is also advantageous from a standpoint of invasiveness.

As described above, in the implant device 1 and the implant assembly, the implantable member 40 received from the opening 11M passes through the communication passage 13P and is discharged from the discharge port 13D. This facilitates an operation, as compared with a case where the user implants the implantable member subcutaneously using tweezers or the like. Therefore, it becomes possible to implant the implantable member more conveniently into a living body.

In the above embodiment, the implant device, the implant assembly, and the holder of the present invention have been described. However, the present invention can be appropriately added, modified, and omitted by one skilled in the art within the scope of the technical idea. For example, the configuration, shape, size, and the like of each part of the implant device, the implant assembly, and the holder described in the above embodiment are just exemplary, and other configurations, shapes, sizes, and the like may also be used.

For example, in the above embodiment, the example in which the holder 20 holding the implantable member 40 is attached to the opening 11M has been described. However, the user may supply the implantable member 40 to the body part 10 from the opening 11M.

In addition, instead of the holder 20 described in the above embodiment, an SP (Strip Package) packaging or the like may be used.

Further, in the above embodiment, the example in which the holder 20 is detachably attached to the opening 11M of the body part 10 has been described. However, the holder 20 and the body part 10 may be integrated.

Further, in the above embodiment, the example in which the stopper 25 is integrated with the holder 20 has been described. However, the stopper 25 may be provided separately from the holder 20.

Note that the present embodiment may have the following configurations.
(1) An implant device configured to implant an implantable member into a living body, the implant device including:
   a receiving portion having an opening surface having an opening capable of receiving the implantable member, and a support surface configured to face the opening surface in a first direction and to support the implantable member; and
   a discharge portion having a discharge port configured to discharge the implantable member, and a communication passage configured to communicate the discharge port and the opening and extending in a second direction intersecting with the first direction.
(2) The implant device according to the above (1), wherein the receiving portion further has a pair of side surfaces configured to connect the opening surface and the support surface and to face each other in a third direction intersecting with the first direction and the second direction.
(3) The implant device according to the above (2), wherein sizes of the receiving portion in the first direction and the third direction are larger than sizes of the discharge portion in the first direction and the third direction.
(4) The implant device according to the above (3), further including a connecting passage configured to connect the opening and the communication passage, wherein sizes of the connecting passage in the first direction and the third direction gradually decrease from the opening side to the communication passage side.
(5) The implant device according to any one of the above (1) to (4), further including a holder provided in the opening and capable of holding the implantable member, wherein the holder includes an accommodating space configured to accommodate the implantable member, a holding portion configured to hold the implantable member in the accommodating space, and a take-out port through which the implantable member is taken out from the accommodating space.
(6) The implant device according to the above (5), wherein at least a part of a wall of the accommodating space is configured to be deformable toward the take-out port side.
(7) The implant device according to the above (5) or (6), wherein the holding portion includes a first protrusion protruding from an edge of the take-out port toward the take-out port.
(8) The implant device according to any one of the above (5) to (7), wherein the holding portion includes a second protrusion protruding from a wall of the accommodating space toward the take-out port.
(9) The implant device according to any one of the above (5) to (8), further including a stopper provided between the opening surface and the support surface and arranged at a position facing the discharge port with the take-out port interposed between the position and the discharge port.
(10) The implant device according to the above (9), wherein the stopper is integrated with the holder.
(11) The implant device according to any one of the above (5) to (10), wherein the holder is configured to be detachably attached to the opening.
(12) The implant device according to the above (11), wherein the holder has a groove capable of fitting to an edge of the opening.
(13) The implant device according to the above (11) or (12), wherein the holder further includes a falling-off prevention portion having a predetermined length between the opening surface and the support surface.
(14) The implant device according to any one of the above (1) to (13), further including a rod part configured so that at least a tip of the rod part is insertable into a part of the communication passage via a gap between the opening surface and the support surface.
(15) The implant device according to the above (14), wherein the rod part further includes a regulating portion configured to regulate a position in the second direction of the tip inserted into the communication passage.
(16) The implant device according to any one of the above (1) to (15), wherein a surface of the implantable member is provided with fine irregularities.
(17) The implant device according to any one of the above (1) to (16), wherein the implantable member is a fragile member.
(18) The implant device according to any one of the above (1) to (17), wherein the implantable member has a cancer cell capturing function.
(19) An implant assembly including the implant device according to any one of the above (1) to (18), and a skin incision instrument having a sharp tip and scales provided at predetermined intervals from the tip.
(20) A holder that is attachable to an implant device configured to implant an implantable member into a living body, the holder including:
   an accommodating space configured to accommodate the implantable member;
   a holding portion configured to hold the implantable member in the accommodating space; and
   a take-out port through which the implantable member is taken out from the accommodation space.

## Claims

1. An implant device configured to implant an implantable member into a living body, the implant device comprising:
a receiving portion having an opening surface having an opening capable of receiving the implantable member, and a support surface configured to face the opening surface in a first direction and to support the implantable member; and
a discharge portion having a discharge port configured to discharge the implantable member, and a communication passage configured to communicate the discharge port and the opening and extending in a second direction intersecting with the first direction.

2. The implant device according to claim 1, wherein the receiving portion further has a pair of side surfaces configured to connect the opening surface and the support surface and to face each other in a third direction intersecting with the first direction and the second direction.

3. The implant device according to claim 2, wherein sizes of the receiving portion in the first direction and the third direction are larger than sizes of the discharge portion in the first direction and the third direction.

4. The implant device according to claim 3, further comprising a connecting passage configured to connect the opening and the communication passage,
wherein sizes of the connecting passage in the first direction and the third direction gradually decrease from the opening side to the communication passage side.

5. The implant device according to claim 1, further comprising a holder provided in the opening and capable of holding the implantable member,
wherein the holder comprises an accommodating space configured to accommodate the implantable member, a holding portion configured to hold the implantable member in the accommodating space, and a take-out port through which the implantable member is taken out from the accommodating space.

6. The implant device according to claim 5, wherein at least a part of a wall of the accommodating space is configured to be deformable toward the take-out port side.

7. The implant device according to claim 5, wherein the holding portion comprises a first protrusion protruding from an edge of the take-out port toward the take-out port.

8. The implant device according to claim 5, wherein the holding portion comprises a second protrusion protruding from a wall of the accommodating space toward the take-out port.

9. The implant device according to claim 5, further comprising a stopper provided between the opening surface and the support surface and arranged at a position facing the discharge port with the take-out port interposed between the position and the discharge port.

10. The implant device according to claim 9, wherein the stopper is integrated with the holder.

11. The implant device according to claim 5, wherein the holder is configured to be detachably attached to the opening.

12. The implant device according to claim 11, wherein the holder has a groove capable of fitting to an edge of the opening.

13. The implant device according to claim 11, wherein the holder further comprises a falling-off prevention portion having a predetermined length between the opening surface and the support surface.

14. The implant device according to claim 1, further comprising a rod part configured so that at least a tip of the rod part is insertable into a part of the communication passage via a gap between the opening surface and the support surface.

15. The implant device according to claim 14, wherein the rod part further comprises a regulating portion configured to regulate a position in the second direction of the tip inserted into the communication passage.

16. The implant device according to claim 1, wherein a surface of the implantable member is provided with fine irregularities.

17. The implant device according to claim 1, wherein the implantable member is a fragile member.

18. The implant device according to claim 1, wherein the implantable member has a cancer cell capturing function.

19. An implant assembly comprising:
the implant device according to any one of claims 1 to 18; and
a skin incision instrument having a sharp tip and scales provided at predetermined intervals from the tip.

20. A holder that is attachable to an implant device configured to implant an implantable member into a living body, the holder comprising:
an accommodating space configured to accommodate the implantable member;
a holding portion configured to hold the implantable member in the accommodating space; and
a take-out port through which the implantable member is taken out from the accommodation space.
